# EUROPEAN PATENT APPLICATION

(11) **EP 4 410 944 A1**
(43) Date of publication of application: **07.08.2024**
(21) Application number: 23154250.7
(22) Date of filing: 31.01.2023
(51) Int. Cl.: C12M 1/00, C12M 3/00, C12M 1/06, C12M 1/02

(54) **REACTOR FOR CULTIVATING CELLS OR MULTICELLULAR MICROORGANISMS**

(71) Applicant: The Cultivated B. GmbH, 69124 Heidelberg (DE)
(72) Inventor:
(74) Representative: Kudla, Karsten

(57) **Abstract**

The invention relates to a reactor for cultivating cells or multicellular microorganisms comprising a tank (3) with a double jacket (9), wherein the double jacket (9) encloses a jacket space (11), wherein at least one spirally circumferential channel (13) is formed in the jacket space (11), the spirally circumferential channel (13) being laterally bounded by inclined walls (15), the inclined walls (15) having a slope of 40° 90° with respect to the horizontal. The invention further relates to a process for cultivating cells or multicellular microorganisms using the reactor (1) and to a process for producing the tank (3).

## Description

The invention relates to a reactor for cultivating cells or multicellular microorganisms comprising a tank with a double jacket and a stirrer, wherein the double jacket encloses a jacket space.

Tanks with a double jacket are a common apparatus for carrying out reactions, which usually are carried out batchwise or semi-batchwise and which need cooling or heating of the contents during the reaction. Besides a double jacket, it is also known to apply cooling coils or heating coils on the outer surface of the tank.

Particularly if used for cultivating microorganisms and cell cultures used in applications such as biotechnology, pharmaceutical and food industries, specific conditions concerning nutrient distribution and availability as well as optimal and homogeneous temperatures are needed for optimal growth of the microorganisms or cells. Current solutions usually are economically unattractive due to specific designs for microorganisms and cell cultures, respectively, as these require specialized bioreactors or impellers as disclosed for example in US-A 2020/0318054 for different uses, depending on the cultured cells or microorganisms.

Further cost factors are single use options as described for example in US-A 2012/0028234 or EP-B 3 240884 for bioreactors or the alternative resource-intensive sterilizing procedures between uses.

Another challenge is the temperature control system, which usually uses a heat blanket as described for example in US-A 2019/0330585, or water-jackets connected to the heating system installed around the bioreactor tank. This approach for example is described in US-B 7,122,371, CA-A 2761251 or US-B 7,264926. However, the current solutions provide suboptimal and comparably ineffective heat transfer into and homogeneous regulation within the tank due to energy transfer between different materials and potential air sockets between jacket or blanket and outer wall of the tank. Furthermore, maintenance of such systems requires chemical based and, thus, again resource intensive and environmentally hazardous methods for decontamination of water-based temperature-regulation equipment if used repeatedly.

Furthermore, the current state of the art in bioreactor technology applies sampling systems to analyze nutrient supply within the culture medium. This requires intermittent sample extraction from the active bioreactor as described for example in CN-A 113999767, which either necessitates further complex accessories on the bioreactor or direct sampling. However, this approach includes a contamination risk during the extraction process and only allows spot checks concerning current nutrient requirements in the culture medium.

Therefore, it is an object of the present invention to provide a reactor for cultivating cells or multicellular microorganisms comprising a tank with a double jacket and a stirrer, which shows a significant improvement of (environmental) sustainability.

These objects are achieved by a reactor for cultivating cells or multicellular microorganisms comprising a tank with a double jacket, wherein the double jacket encloses a jacket space, wherein at least one spirally circumferential channel is formed in the jacket space, the spirally circumferential channel being laterally bounded by inclined walls, the inclined walls having a slope of 40° to 90° with respect to the horizontal.

By the inventive design of the tank, a uniform flow of the heating medium or cooling medium (in the following also denoted as "tempering medium") can be realized and the contents of the tank can be tempered uniformly. Further, in difference to present reactors for cultivating cells or microorganisms, no extra temperature control spiral or homeothermic blanket or jacket is required. Due to the inventive double jacket with the at least one spirally circumferential channel is formed in the jacket space, which is laterally bounded by inclined walls, a homogenous and direct heat exchange with a tempering medium through only a single wall is ensured. For tempering the contents in the tank, the tempering medium usually is introduced via a first opening, flows through the spirally circumferential channel and is discharged via a second opening. Preferably, the first opening is at the top of the tank and the second opening is at the bottom, so that the tempering medium flows downwards along the spirally circumferential channel. By using the inventive reactor, the energy loss can be significantly reduced and a contamination risk also is eliminated. Further, the inner wall may be made of a uniform material and/or only one material in contrast to single wall reactors where the wall may be made of several different materials, and heat exchange air pockets are prevented due to the specific construction of the reactor.

It is a further advantage of the reactor comprising the tank with double jacket enclosing a jacket space, in which at least one spirally circumferential channel being laterally bounded by inclined walls having a slope of 40° to 90° is formed with respect to the horizontal is formed, that the whole tank can be produced by a 3D-printing process. Suitable 3D-printing processes particularly are such, in which a powdery material is sintered by energy input, thereby forming the tank. Particularly preferably, the tank is made of a metal and, thus, the 3D-printing process is such a process which allows for forming a three-dimensional object from the metal. Particularly preferably, the 3D-printing process used for forming the tank is a laser powder bed fusion technology. After finishing the 3D-printing process, non-sintered powder may be removed from spirally circumferential channel through the openings for introducing and removing the tempering medium by shaking and/or vibrating. Alternatively, the powder may be removed for example by blowing it out with a pressurized gas, particularly pressurized air. For easier removal further holes through which the powder can exit may be provided, preferably at least one further hole in each row of the spiral. After removing the powder, the additional holes will be sealed, for example by blind flanges.

By the 3D-printing process, the tank is formed including the double jacket, wherein the double jacket encloses a jacket space and at least one spirally circumferential channel is formed in the jacket space, the spirally circumferential channel being laterally bounded by inclined walls. To be printable by the 3D-printing process, the inclined walls have a slope of 40° to 90° with respect to the horizontal. If the walls of the reactor have a vertical orientation, the angle of the inclined walls is less than 90°, preferably less than 80°.

According to the invention, the inclined walls may have an inclination with a decline from the inner wall to the outer wall of the double jacket. Alternatively, it is also possible that the inclined walls have a decline from the outer wall to the inner wall of the double jacket.

By producing the tank in a 3D-printing process, it is possible to easily adapt the shape and the size of the tank reactor to various uses. Further, as the tank can be adapted to a specific use, production of the tank does not have the problems of current technologies regarding consumption of raw materials and the economic challenges due to specialized and single-use bioreactors.

The tank may be made of any suitable material, particularly any suitable material which can be processed in a 3D-printing process. Preferably, the tank and the lid are made of a metal and particularly preferably of stainless steel. The material used for fabricating the tank thereby depends on the intended use of the tank reactor. Particularly if used in food processing, the tank is made of stainless steel.

Besides producing the tank in a 3D-printing process, the tank also may be produced for example by a casting process or by injection molding. In this case, the tank may be made from glass, ceramic or a plastic material. If a weldable material is used, for example a metal or a thermoplastic polymer, the tank also may be produced by a welding process. However, any other manufacturing process which allows production of the tank also may be used.

As the tank reactor produced by the 3D-printing process usually has not a smooth inner surface, it is preferred to treat the surface before using the tank, for example by polishing to achieve a smooth surface or by applying a coating. Such a coating for example may be a coating that prevents cell adhesion to the tank walls. If a coating is used, any coating may be used which does not affect the processes to be carried out in the tank. Particularly for cultivating cells or microorganisms, it is important that the coating does not free any poisonous substances. Therefore, it is particularly preferred to treat the surface mechanically, for example by polishing, but not to apply any coating on the surface.

Particularly if the reactor is used for cultivating cells or multicellular microorganisms which need a specific atmosphere, it is preferred that the tank comprises a gastight closable lid. By the gastight closable lid it can be avoided that material can leave the reactor unintendedly at the top opening and, further, it also can be prohibited that the contents in the reactor may unintendedly come into contact with the outer atmosphere, particularly with air, or, if the tank reactor is used in a different atmosphere, with this atmosphere.

As the lid must fit to the tank, it is preferred, to also produce the lid in the 3D printing process. It further is preferred to produce the lid from the same material as the tank. In this case, it is particularly preferred to produce the tank and the lid in the same process.

For feeding material into the tank, for example culture media or nutrient solutions, for taking samples, or for providing sensors, the lid preferably comprises openings for at least one of a sensor, an inlet and an outlet. For taking samples or withdrawing material from the reactor, it is further preferred to use a dip tube, which is guided through a respective opening in the lid. The position of the inlet of the dip tube preferably is selected such that the sample can be taken from a respective position in the tank, for example at the bottom, from the middle or at the top. Suitable sensors, which may be used, for example are temperature sensors, pressure sensors, pH-sensors, oxygen sensors, optical sensors or sensors for media components (e.g. amino acids, carbohydrates, metabolites, etc.). Suitable sensors may be analog sensors or digital sensors, which can measure biochemical factors and/or waste products of a biochemical process carried out in the reactor and which may operate continuously or discrete. Using such sensors has the additional advantage that taking spot samples will be unnecessary and reduce delayed nutrient regulation, although manual sampling is still possible if required.

After having finished a cultivating process in the tank, usually the contents must be withdrawn. For this purpose, the contents may be withdrawn through the lid by a dip tube which ends close to the bottom of the tank. However, preferably, the tank comprises a material outlet at the bottom. During normal operation, the material outlet at the bottom is closed, for example by a valve, a plug or a lock. For withdrawing the material, the material outlet is opened and the contents of the tank may flow out of the tank by force of gravity or, alternatively, by applying a vacuum to the material outlet, for example by a vacuum pump. To avoid that the contents of the tank flow through the pump, in this case it is preferred to connect a buffer container to the material outlet and set the vacuum to the buffer container, so that the contents of the tank are drawn into the buffer container.

By the separate openings for taking samples, feeding material into the reactor and the sensors, it is not necessary to either open the lid or to take the samples through the material outlet. Thus, it is possible to take spot samples with less contamination risks and prevent delayed nutrient regulation.

For fixing the lid on the tank, any suitable method can be used. For fixing the lid, it may be possible to provide an external thread on the lid and an internal thread on the tank and to screw the lid on the tank. If the lid is provided with a sleeve it alternatively is possible to provide the internal thread in the sleeve and the external thread on the outer wall of the tank.

However, to allow a quick opening or closing, it is preferred to lock the tank and the lid by snap locks, latches or spring locks.

Further, particularly if the tank shall be closed tightly to avoid gas or liquid leaving the tank or entering the tank at the lid, it is preferred to apply a seal between the lid and the tank. Suitable seals for example are O-rings, sealing cords or gaskets. Further, if the lid comprises openings for inlets, outlets or sensors, further suitable seals are provided at each opening for a tight closing of the tank.

For using the tank repeatedly, it is necessary to clean the tank after use and particularly to sterilize the tank before the next use. For this purpose, the tank usually is treated by autoclave. This either may be carried out by putting the tank and the lid into an autoclave or, alternatively, to close the lid and treat the interior of the tank with high pressure vapor. As the tank and the lid can be easily treated by autoclave, either by placing the tank and the lid into an autoclave or alternatively by feeding high pressure water vapor into the tank, the vapor thus having a temperature above any temperature cells or microorganisms may survive, the tank allows for less environmentally hazardous and costly decontamination methods.

Further, besides treating the reactor by autoclave, it also may be possible to use the reactor as an autoclave itself. In this case water will be filled into the reactor and by using a suitable tempering medium, for example superheated steam or a hot gas, the water is heated to the boiling point. Further, a pressure may be applied to the interior of the reactor to increase the boiling point of the water.

Depending on the pressure of the process to be carried out in the tank and the pressure for treatment by autoclave, the wall thickness is selected. The wall thickness must be such that the tank withstands the pressures applied. As the second wall of the double jacket and particularly the inclined walls of the channel in the jacket space also stabilize the tank, the inner wall may be less thick than in a tank without the double jacket and the inclined walls. Thereby the thickness of the inner wall also depends on the distance of adjacent inner walls.

That the inner wall may be thinner than in a tank without the double jacket and the inclined walls has the additional advantage that the heat transfer into the tank is further improved, and, for this reason, the energy consumption for heating or cooling the tank may be reduced.

Depending on the process for which the reactor shall be used, the tank may have a nominal volume in a range between 0.5 and 25,000 L, preferably the tank has a nominal volume in a range between 1 and 1000 L, more preferred in a range between 1 and 100 L, and particularly in a range between 1 and 10 L.

Depending on the cells or multicellular microorganisms to be cultivated in the reactor, it may be necessary to provide a stirrer. As for different cultures different stirrers may be used, it is further preferred that the stirrer is changeable. For easily changing the stirrer, the stirrer preferably is releasably connected to a drive. For this purpose, the stirrer may be connected to the drive by screwing, bayonet fastening or a magnet, wherein a connection by bayonet fastening or a magnet is preferred.

In the context of the present invention, the term "cells" comprises animal cells, human cells, plant cells, bacterial cells, protozoal cells, archaebacterial cells, fungal cells, and also monocellular microorganisms. Multicellular microorganisms for example are fungi, plants, algae or microalgae as long as not monocellular. Preferably, the inventive reactor is used for the cultivation of cells, like mammalian cells, avian cells or fish cells, bacteria, fungi, and other microorganisms, plants, algae or microalgae. Further, the reactor also may be used for slow cooking methods, yeast fermentation for dough or for alcoholic beverages, for example for beer fermentation processes.

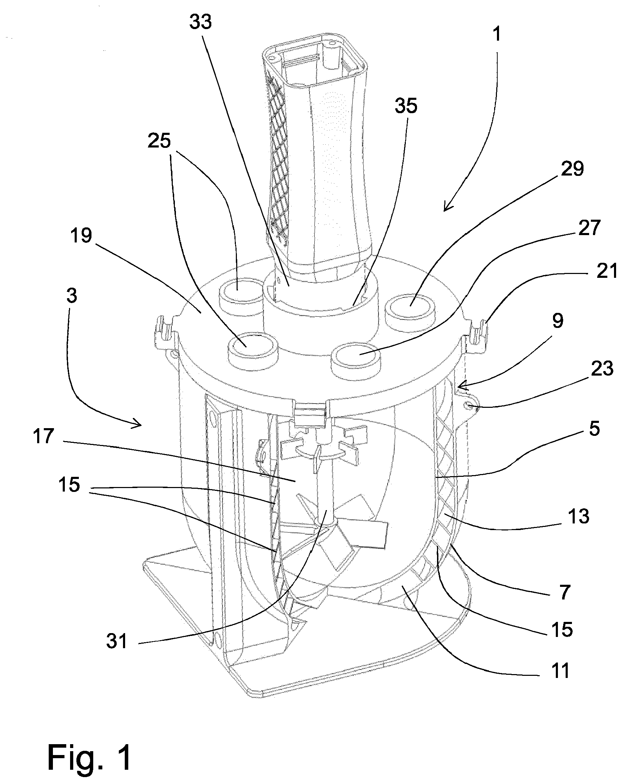

An embodiment of the invention is shown in the figure and described in more detail in the following description.

The only figure shows a three-dimensional view of an inventive reactor with a cutout for showing the double jacket with the circumferential channel.

A reactor 1 for cultivating cells or multicellular microorganisms comprises a tank 3. The tank 3 comprises an inner wall 5 and an outer wall 7, thereby forming a double jacket 9, which encloses a jacket space 11. For homogeneously heating or cooling the interior of the tank 3, at least one spirally circumferential channel 13 is formed in the jacket space 11.

The at least one spirally circumferential channel 13 is laterally bounded by inclined walls 15. According to the invention, the inclined walls 15 have a slope of 40° to 90° with respect to the horizontal.

For heating or cooling the interior 17 of the tank 3, a heating medium or a cooling medium flows through the at least one spirally circumferential channel 13. The heating or cooling medium may be fed at the bottom and withdrawn at the top of the tank 3, or, alternatively, fed at the top and withdrawn at the bottom of the tank 3. If the tank 3 comprises at least two spirally circumferential channels 13, it is also possible to feed the heating or cooling medium in at least one spirally circumferential channel 13 at the bottom and into at least one spirally circumferential channel 13 at the top, and withdraw the heating medium or cooling medium, which is fed at the bottom, at the top and the heating or cooling medium, which is fed at the top, at the bottom. In this case it is particularly preferred, if the heating medium or cooling medium flows in countercurrent in directly adjacent spirally circumferential channels 13.

The inclined walls 15 may have an inclination as shown in the figure with a decline from the inner wall 5 to the outer wall 7. Alternatively, it is also possible that the inclined walls have a decline from the outer wall 7 to the inner wall 5. It is only important for a 3D-prinability of the tank that the walls by which the spirally circumferential channel 13 is laterally bounded have an inclination and are not horizontal.

The reactor 1 may further comprise a lid 19. The tank 3 can be tightly closed by the lid 19 so that the interior 17 of the tank 3 is sealed off from the environment and neither air or gases of any other atmosphere can unintendedly flow into the reactor and, on the other hand, no gases and/or liquids contained in the interior 17 of the tank may leak from the tank 3 to the surrounding atmosphere. This is particularly important, if during the cultivation process in the reactor 1 any toxic or environmentally hazardous gases are produced.

Further, tightly closing the tank 3 also allows for operating a cultivating process in the tank 3 under pressure or, in the alternative, at a pressure below ambient pressure. Additionally, by closing the tank 3 gastightly with the lid 19, it is also possible to treat the interior 17 with high pressure vapor for sterilizing.

For gastight closing of the tank 3, it is preferred to lock the lid 19 to the tank 3. Locking for example may be carried out by screwing the lid 19 to the tank 3. For screwing the lid 19 to the tank 3, it is for example possible to provide threads on the lid 19 and the tank 3, or alternatively to use screws. If screws are used, the tank may comprise a flange and holes are provided in the lid and the flange through which screws are guided and to fix the screws by corresponding nuts, or alternatively, holes are provided in either the lid or the tank and threads in the tank or the lid and screws are guided through the holes and screwed into the threads. Besides locking by screwing the lid 19 to the tank 3, alternatively, the lid 19 may be locked by snap locks, latches or spring locks.

For locking, the lid may comprise hooks 21 as shown here and corresponding clips (not shown here) may be fixed in openings 23 on the outer wall 7 of the tank 3. For closing, the clips are clamped in the hooks 21.

A gastight closing of the tank 3 further necessitates a seal between the tank 3 and the lid 19. For this purpose, any suitable seal can be used, for example an O-ring, a sealing cord or a gasket.

For feeding components into the interior 17 of the tank 3 after the lid 19 is closed, the lid preferably comprises openings 25 as shown here. The openings 25 which are used for feeding components into the interior 17 of the tank 3 may be connected for example with suitable tubes or pipes, through which the components may be dosed into the tank 3. For dosing the required amounts of the components, it is for example possible to use dosing pumps. Further, it also may be possible to connect the tank 3 to a balance and weigh the amount of the components fed into the tank 3 through the openings 25. Besides the openings 25 for feeding components into the tank, the lid may further be equipped with openings 27 for sensors and openings 29 for withdrawing material from the interior 17 of the tank, for example for taking samples. For taking samples or withdrawing material from the interior 17 of the tank, via an opening in the lid, for example a dip tube may be used which is guided through a respective opening 29. The depth, the dip tube extends into the tank 3 thereby depends on the position at which a sample shall be taken. If the material is withdrawn from the tank via a dip tube, the dip tube extends into the tank 3 down to the bottom. Besides a dip tube or in addition, the tank 3 may comprise a material outlet at the bottom, which is not shown here.

The number of the openings 25, 27, 29 for feeding components into the tank 3, for sensors and for withdrawing material from the tank 3, respectively depend on the number of different components, the number of sensors used for the process and the number of withdrawal points needed. Thus, if for example only one component is to be fed into the tank 3 or only one sensor is used, only one opening 25 for feeding components or only one opening 27 for a sensor may be provided. However, particularly for allowing to use the reactor 1 for different processes, it is particularly preferred that the openings 25, 27, 29 all have the same shape and, thus, can be used for any purpose. In this case, adapters may be used for the different applications. Further, if there are more openings than needed, the openings 25, 27, 29, which are not used, may be closed by suitable closings.

As in processes for cultivating cells or multicellular microorganisms the liquid contents of the reactor 1 usually must be agitated, the reactor 1 further comprises a stirrer 31. The stirrer 31 is connected to a drive 33, which in the embodiment shown in the figure is arranged centrally in the lid 19. Beside the central arrangement shown here, it is also possible to arrange the drive of the motor eccentric. In this case the respective opening in the lid 19 for mounting the drive 33 is at the respective position outside the center of the lid. In this case, one of the openings 25, 27, 29 may be arranged centrally.

If it is intended to use the reactor 1 in different processes, which may need different types of stirrers, it is particularly preferred, if the stirrer 31 is releasably connected to the drive 33. The connection may be realized by screwing the stirrer 31 to the drive 33, by a magnetic connection or a bayonet fastening.

To avoid that the drive rotates in its seat in the lid 19, the drive may be equipped with grooves and the seat with tongues or, alternatively as shown here, the seat with grooves 35 and the drive with tongues.

## Claims

1. A reactor for cultivating cells or multicellular microorganisms comprising a tank (3) with a double jacket (9), wherein the double jacket (9) encloses a jacket space (11), wherein at least one spirally circumferential channel (13) is formed in the jacket space (11), the spirally circumferential channel (13) being laterally bounded by inclined walls (15), the inclined walls (15) having a slope of 40° to 90° with respect to the horizontal.

2. The reactor according to claim 1, wherein the (3) tank comprises a gastight closable lid (19).

3. The reactor according to claim 2, wherein the lid (19) comprises openings (25, 27, 29) for at least one of a sensor, an inlet and an outlet.

4. The reactor according to claim 2 or 3, wherein the tank (3) and the lid (19) are locked by snap locks, latches or spring locks.

5. The reactor according to any of claims 1 to 4, wherein the tank (3) and the lid (19) are made of optionally coated stainless steel.

6. The reactor according to any of claims 1 to 5, wherein the reactor (1) comprises a stirrer (31).

7. The reactor according to claim 6, wherein the stirrer (31) is releasably connected to a drive (33).

8. The reactor according to any of claims 1 to 7, wherein the stirrer (31) is connected to the drive (33) by a bayonet fastening or a magnet.

9. A process for cultivating cells or multicellular microorganisms using a reactor (1) according to any of claims 1 to 8.

10. A process for producing the tank (3), wherein the tank (3) is produced by a 3D-printing process,

11. The process according to claim 10, wherein the 3D-printing process is a laser powder bed fusion technology.
